# EUROPEAN PATENT SPECIFICATION

(11) **EP 3 706 714 B1**
(45) Date of publication and mention of the grant of the patent: **14.09.2022**
(21) Application number: 18795526.5
(22) Date of filing: 29.10.2018
(51) Int. Cl.: A61K 31/17, A61K 31/198, C08L 5/16, A61K 9/00, C08B 37/16, A61K 47/69

(54) **N-ACETYLCYSTEINE AND UREA-BASED FORMULATION FOR THE TREATMENT OF DERMATOLOGICAL DISORDERS**
N-ACETYLCYSTEIN- UND HARNSTOFFBASIERTE FORMULIERUNG ZUR BEHANDLUNG VON DERMATOLOGISCHEN ERKRANKUNGEN
FORMULATION À BASE DE N-ACÉTYLCYSTÉINE ET D'URÉE POUR LE TRAITEMENT DE TROUBLES DERMATOLOGIQUES

(30) Priority: 06.11.2017 IT 201700125963
(43) Date of publication of application: 16.09.2020
(73) Proprietor: Dermalena Di Calderan Andrea, 30027 San Donà di Piave (VE) (IT); Calderan, Alessandro, 30027 San Donà di Piave (VE) (IT)
(72) Inventor: CALDERAN, Alessandro, 30027 San Donà di Piave (VE) (IT)
(74) Representative: Ferreccio, Rinaldo
(86) International application number: PCT/EP2018/079535
(87) International publication number: WO 2019/086366

(56) References cited:
- CN-A- 104 225 583
- Ja Kim: "Topical use of N-acetylcysteine for reduction of skin reaction to radiation therapy.", , 1 January 1983 (1983-01-01), pages 86-92, XP055545173, Retrieved from the Internet: URL:http://www.ncbi.nlm.nih.gov/pubmed/640 3989 [retrieved on 2019-01-21]
- JOSÉ PARDO MASFERRER ET AL: "Prophylaxis with a cream containing urea reduces the incidence and severity of radio-induced dermatitis", CLINICAL AND TRANSLATIONAL ONCOLOGY, vol. 12, no. 1, 1 January 2010 (2010-01-01), pages 43-48, XP055543268, Italy, Spain ISSN: 1699-048X, DOI: 10.1007/s12094-010-0465-0
- JUERGEN LADEMANN ET AL: "Efficient Prevention Strategy against the Development of a Palmar-Plantar Erythrodysesthesia during Chemotherapy", SKIN PHARMACOLOGY AND PHYSIOLOGY: JOURNAL OF PHARMACOLOGICAL ANDBIOPHYSICAL RESEARCH, vol. 27, no. 2, 1 January 2014 (2014-01-01), pages 66-70, XP055543039, CH ISSN: 1660-5527, DOI: 10.1159/000351801
- ADRIANA BASSOTTI ET AL: "Successful Treatment with Topical N-Acetylcysteine in Urea in Five Children with Congenital Lamellar Ichthyosis : Successful Treatment with Topical N-Acetylcysteine", PEDIATRIC DERMATOLOGY., vol. 28, no. 4, 1 July 2011 (2011-07-01), pages 451-455, XP055487479, US ISSN: 0736-8046, DOI: 10.1111/j.1525-1470.2011.01375.x
- LUIS NOGUEIRAS-NIETO ET AL: "Thermogelling hydrogels of cyclodextrin/poloxamer polypseudorotaxanes as aqueous-based nail lacquers: Application to the delivery of triamcinolone acetonide and ciclopirox olamine", EUROPEAN JOURNAL OF PHARMACEUTICS AND BIOPHARMACEUTICS, vol. 83, no. 3, 1 April 2013 (2013-04-01), pages 370-377, XP055136313, ISSN: 0939-6411, DOI: 10.1016/j.ejpb.2012.11.004

## Description

### Technical Field

The present invention relates to the technical field of the pharmaceutical industry.

In particular, the invention relates to a formulation for topical application for use in the prevention and/or treatment of dermatological disorders.

### Background Art

It is known that skin, being the largest organ of the human body, is subjected to numerous stress sources contributing to the onset of dermatological disorders of different nature.

Among these, for example, it is known that often patients suffering from cancer and undergoing a chemotherapeutic treatment, including the administration of the drug doxorubicin, or analogues thereof, or of many other chemotherapeutic drugs, even biological, can develop palmar-plantar erythrodysesthesia, also known in oncology as hand-foot syndrome.

After its onset, generally, with a tingling and burning sensation on the palms of the hands and, less frequently, on the soles of the feet, palmar-plantar erythrodysesthesia causes intense erythema and swelling in a few days.

Subsequently, vesicles and ulcerations appear, more frequently on areas subjected to pressure, with severe pain and/or severe discomfort that make it impossible for the patient to work or to carry out daily activities.

Desquamation and intense erythema make the affected areas similar to second-degree superficial burns.

According to some studies, the reason whereby this disorder develops on the skin of the soles of the feet and the palms of the hands would be correlated with the high presence of sweat glands and the thickness of the stratum corneum in these regions (Ya-Xian et al. Arch Dermatol Res 1999; 291: 555-559).

In fact, the stratum corneum in the palmar-plantar region is about 10 times thicker than that of any other skin area of the body and can quickly accumulate important amounts of chemotherapeutic substances, such as doxorubicin, administered during the chemotherapeutic treatment.

Lademann et al., (Efficient prevention strategy against the development of a palmar-plantar erythrodysesthesia during chemiotherapy, Skin Pharmacol Physiol 2014, 27; 66-70) endorse that the presence of a high concentration of doxorubicin accumulated in the stratum corneum of hands and feet generates free radicals that, transported by the sweat produced by the sweat glands, spread and reach the surface of the skin, damaging the skin tissue and causing the aforementioned symptoms.

No standard treatments have been established for the treatment of this skin disorder so far. In many cases, the chemotherapy treatment must be reduced or, in cases of more aggressive symptoms, should be discontinued (Lorusso et al., Ann Oncol 2007; 18: 1159-1164).

Lademann proposes the topical application of an oil-in-water formulation containing microparticles consisting of a mixture of silica and *Hippospongia communis* (sponge) and loaded with antioxidants such as green tea extract, green coffee extract, seed extract of *Pongamia pinnata* and *Angelica* extract.

It is also known that dermatological disorders occur even in the case in which the patient is subjected to radiotherapy cycles to combat cancer spread.

In fact, the skin is affected by repeated exposure to the 5-10 MeV energy beam which is typically used in this oncology technique, and therefore serious skin reactions are also frequent.

The most common effects are: xerosis (extreme dehydration accompanied by itching and desquamation), radiodermatitis (inflammation in the treated areas with redness, irritation, desquamation, pain and blisters), pigmentation changes (white, dark spots and/or telangiectasias) and alopecia.

In addition, the sebaceous and sweat glands in the treated area can be damaged, with impairment of their secretion functions and consequent dehydration and a more fragile and sensitive skin.

Since such radiation causes the formation of reactive oxygen species (ROS) in cells and tissues subjected to such treatment, the use of antioxidant compounds to reduce their inflammation and damage is known.

Kim et al., (Seminars in Oncology, vol 10, n.1, Suppl 1 (March) 1983) discloses the use of a gauze soaked in a 10% N-acetylcysteine solution applied to the area affected by radiation for about 15 minutes before each daily radiotherapy treatment.

Patent application EP 2 583 682 discloses an antioxidant composition to be applied topically, comprising N-acetylcysteine and galactomannans for use in the therapeutic or prophylactic treatment of a skin disorder or a skin condition caused by exposure to sunlight, chemical agents, radiotherapy or chemotherapy.

Tascilar et al., (N-acetylcysteine attenuates the deleterious effects of radiation therapy on incisional wound healing in rats, HIPPOKRATIA 2014, 18, 1: 17-23) evaluates the effect of oral and intra-peritoneal administration of N-acetylcysteine on histological, biochemical and mechanical parameters of the healing of an abdominal incisional wound in a mouse model subjected to radiation, highlighting the effectiveness of such antioxidant compound.

Another example of skin disorder is the one related to the Sudeck syndrome, also known as complex regional pain syndrome (CRPS).

This pathology often arises as a result of traumas (sprains, fractures), followed by incorrect reductive manoeuvres, including plastered immobilization in anti-physiological positions, prolonged immobility, absence of load, insufficient or failed reduction in the case of fracture or dislocation.

Important triggers include infections (infected fractures, osteomyelitis, osteoarthritis), burns and frostbite, secondary lesions from chemical or physical agents (X rays, electricity), organic or functional vasculopathies, nervous system disorders.

This pathology also involves dermatological disorders, including vasomotor changes, skin and subcutaneous edema, trophic changes of skin, cutaneous appendages and subcutaneous tissue.

The most common treatment for these symptoms is mainly based on an analgesic pharmacological therapy associated with a progressive and constant mobilization of the affected extremities, to maintain a good joint mobility and promote an early muscle recovery at a later stage.

According to Perez et al., (The treatment of complex regional pain syndrome type I with free radical scavengers: a randomized controlled study, R.S.G.M. Perez et al. / Pain 102 (2003) 297-307), a further treatment strategy lies in the administration of antioxidant agents, as this syndrome appears to be induced by a strong inflammatory response to tissue lesions mediated by an excessive production of toxic oxygen radicals; the tested antioxidant agents were 50% DMSO applied topically and N-acetylcysteine taken orally. The results show that both compounds are equally efficient in the treatment of this disease.

Another dermatological disorder that negatively affects the living conditions of patients with diabetes is the diabetic foot syndrome.

The hyperglycemia associated with diabetes is responsible for a series of morphological and functional alterations in the dermis, inducing a biochemical alteration of collagen that becomes harder and resistant. There is therefore a progressive thickening and hardening of the dermis which becomes more compact and rigid.

In addition to damage to the collagen fibers, structural and irreversible anomalies in the elastic fibers also occur, which tend to disappear, thus compromising the elasticity of dermis.

Furthermore, hyperglycemia involves the destruction of the anchoring fibers that fix dermis to epidermis, thus predisposing the epidermis to the onset of easy abrasions even upon a minimum mechanical or physical stress.

As a consequence, the dermis of the diabetic subject has a poor resistance to physical stress and consequently, where the skin is thinner, abrasions and chaps are formed after a minimum rubbing, while where it is thicker, as at the plantar level, callous thickening is observed.

Patent application US 2016/0338993 relates to a method of treatment of a chronic wound, preferably a skin ulcer of a diabetic subject, involving the administration of at least one antioxidant agent selected from, for example, N-acetylcysteine, vitamin A, vitamin C, vitamin E, glutathione, superoxide dismutase, flavonoids and phenols.

Patent application US 2003/0229141 discloses a composition for the topical treatment of skin diseases comprising at least one N-acetyl-amino acid (preferably N-acetylcysteine) and, optionally, also a cosmetic or pharmaceutical agent.

International application WO 97/15283 (Procter & Gamble) relates to a topical composition comprising from 0.01 to 50% of N-acetylcysteine or a derivative thereof, from 0.01 to 0.5% of a substance able to mask the bad smell associated with the application of N-acetylcysteine to the skin, selected from a group of chemical compounds of perfumes, and a cosmetically acceptable vehicle.

The objective technical problem underlying the present invention is that of providing an easily applicable formulation for use in the treatment of dermatological disorders in which free radicals or reactive oxygen species are involved, in particular dermatological disorders characterized by inflammation, dryness, dehydration, erythema, thickening of the dermis and/or desquamation.

### Summary of Invention

The present invention solves the aforementioned problem by providing a formulation for topical application comprising, in percentages by weight of the total weight of the formulation, 0.5%-10% of N-acetylcysteine and 2-30% of urea, and a dermatologically acceptable vehicle, for use in the treatment and/or prevention of a condition selected from the group consisting of dermatological disorders of the Sudeck syndrome, chemotherapy-induced palmar-plantar erythrodysesthesia, dermatological disorders caused by radiotherapy and diabetic foot syndrome.

Preferably, the formulation for the above use comprises 1-4%, more preferably 1.5-3%, of N-acetylcysteine and 5-25%, more preferably 7-20%, of urea.

In one embodiment, the aforementioned N-acetylcysteine is in the form of a complex with beta-cyclodextrins, preferably 2-hydroxypropyl-betacyclodextrins.

Preferably, the aforementioned N-acetylcysteine is of synthetic origin.

In one embodiment, the formulation for the above use comprises in percentages by weight of the total weight of the formulation:

| | |
|---|---|
| Water | 29-65 |
| Urea | 7-20 |
| Cetearyl alcohol | 2.75-8.0 |
| Ethylhexyl palmitate | 4.0-6.0 |
| Glycerin | 3.0-4.0 |
| Lactic acid | 1.8-4.5 |
| N-acetylcysteine | 1.5-3.0 |
| Sodium hydroxide | 1.2-2.8 |
| Glyceryl stearate | 1.0-1.5 |
| Benzyl alcohol | 0.8-0.9 |
| Perfume | 0.6-0.8 |
| Dimethicone | 0.4-0.6 |
| Xanthan gum | 0.3-0.5 |
| Dehydroacetic acid | 0.08-0.1 |
| Lecithin | 0.02-0.03 |
| Tocopherol | 0.004-0.1 |
| Ascorbyl palmitate | 0.003-0.01 |
| Citric acid | 0.0007-0.01 |

In another embodiment, the aforementioned formulation for the above use comprises in percentages by weight of the total weight of the formulation:

| | |
|---|---|
| Water | 29-65 |
| Urea | 7-20 |
| Cetearyl alcohol | 2.75-8.0 |
| Ethylhexyl palmitate | 4.0-6.0 |
| Glycerin | 3.0-4.0 |
| Lactic acid | 1.8-4.5 |
| N-acetylcysteine | 1.5-3.0 |
| 2-Hydroxypropyl-beta-cyclodextrins | 3.5-4.5 |
| Sodium hydroxide | 1.2-2.8 |
| Glyceryl stearate | 1.0-1.5 |
| Benzyl alcohol | 0.8-0.9 |
| Perfume | 0.6-0.8 |
| Dimethicone | 0.4-0.6 |
| Xanthan gum | 0.3-0.5 |
| Dehydroacetic acid | 0.08-0.1 |
| Lecithin | 0.02-0.03 |
| Tocopherol | 0.004-0.1 |
| Ascorbyl palmitate | 0.003-0.01 |
| Citric acid | 0.0007-0.01 |

Preferably, the formulation according to the invention has a pH between 5.5 and 6.5, advantageously equal to about 6.

Preferably, the formulation is in the form of a cream or a lotion.

The formulation according to the present invention comprises, as active ingredients, N-acetylcysteine and urea.

N-acetylcysteine is known to have an antioxidant action, already mentioned previously with reference to the prior art.

According to an advantageous embodiment of the present invention, N-acetylcysteine is used in the formulation of the present invention in a complexed form and encapsulated with beta-cyclodextrins.

In addition to the remarkable specific moisturizing properties of the sugar cyclic molecule, beta-cyclodextrins ensure, due to their known controlled-release properties, a notable increase in activity of the complexed active ingredient and a significant decrease of any irritation problems related to the active ingredient in the free form.

Furthermore, beta-cyclodextrins make the substances incorporated therein more stable and resistant to oxidation, air degradation and temperature, irrespective of whether they are lipo-soluble or water-soluble, thus improving the conservation and handling of the used active substances.

Due to its particular structure, the complex consisting of N-acetylcysteine and beta-cyclodextrins, in particular 2-hydroxypropyl-beta-cyclodextrins, has a further advantage, *i.e*. that of significantly reducing the intense smell typical of N-acetylcysteine and, therefore, making more pleasant the use of this formulation on the skin.

The other active ingredient, *i.e*. urea, reconstitutes the natural moisturizing factor (NMF) of the epidermis, acting as a moisturizer and emollient and, moreover, it displays an important keratolytic activity, causing the exfoliation of the thicker layers of the dermis and thus promoting the thinning of the thickened skin areas.

The present invention will be further described with reference to the following figures and to some embodiments provided for illustrative and not limitative purposes.

### Brief Description of Figures

Figure 1 shows a photograph of a skin lesion, characterized by inflammation, dermatitis and desquamation, of a patient undergoing radiation therapy.
Figure 2 shows a photograph of the skin lesion of Figure 1 after treatment with the formulation of the present invention.
Figures 3A-3F show photographs of the diabetic foot syndrome of a patient with diabetes.
Figures 4A-4C show the photographs of the foot of the same patient of Figures 3A-3F, after treatment with the formulation of the present invention.
Figures 5A-5C show comparative photographs of the foot of a patient with diabetes, before (left) and after (right) treatment with the formulation of the present invention.

### Detailed Description

Unless otherwise specified, the percentages indicated in the present invention are expressed in "percentage by weight" (% w/w) indicating the weight of the substance with respect to the total weight of the formulation.

"Pharmaceutically acceptable salt" includes in the meaning thereof the salts of the compound in question able to maintain or improve the tolerability and/or biocompatibility of the compound they are derived therefrom.

The term "topical application" means an administration by direct contact of the present composition with the site of action for which it is intended, typically the skin and/or mucous membranes, thus exerting an action at a local level.

As indicated above, the N-acetylcysteine and urea-based formulation of the invention acts in different ways.

In particular, N-acetylcysteine re-establishes the correct concentration of glutathione within cells, which results to be reduced in the pathological conditions indicated above; glutathione, in turn, increases the ability of the epidermal cells to counteract the action of free radicals and, therefore, prevents the lesions caused by the accumulation of such radicals.

Furthermore, N-acetylcysteine hydrolyses the disulphide bridges between the skin layers, thus favouring the exfoliation of thicker layers.

A similar action is also characteristic of urea, whose important keratolytic action allows exfoliation of the thicker layers of the dermis and the thinning of the thickened areas of the skin. Furthermore, urea has a moisturizing action promoting the penetration of N-acetylcysteine until the basal cellular layer.

Moreover, the present formulation is suitable for a topical application, as it can be formulated so to be applied onto the skin, for example in the form of a cream or a lotion, allowing the local application in a simple and functional way.

Furthermore, the formulation of the invention allows to obtain a combined and synergistic action of the active ingredients composing it, *i.e*. N-acetylcysteine and urea, in particular exerting an increased and effective reparative function.

The N-acetylcysteine ingredient is present in the formulation of the invention in amounts between 0.5% and 10% w/w, preferably between 1.5% and 3%.

The urea ingredient is present in the formulation of the invention in amounts between 2% and 30% w/w, preferably 5-25%, conveniently 7-20%.

In one embodiment of the invention, the formulation comprises 3% of N-acetylcysteine and 20% of urea; this combination of active agents allows the use of the formulation for preventive and therapeutic purposes, in which it is applied topically to the affected dermatological area, which is, for example, swollen and/or chapped.

In another embodiment of the present invention, the formulation is a so-called "maintenance formulation", used after completing the treatment with the preventive and therapeutic formulation described above, to keep the treated area moisturized, elastic and compact. This composition, being conceived for a maintenance activity of the results obtained with the therapeutic formulation above, contains N-acetylcysteine and urea in smaller amounts than those in the therapeutic formulation.

In fact, this maintenance formulation comprises 1.5% of N-acetylcysteine and 7% of urea and is applied topically to the area previously treated with the therapeutic formulation.

According to another embodiment of the invention, N-acetylcysteine is complexed with beta-cyclodextrins, cyclic oligosaccharides produced by breaking the starch molecule by means of the enzyme cyclodextrin-glycosyl-transferase (CGT).

As described above, the presence of beta-cyclodextrins not only guarantees a better stability of the product, but also brings about a strong neutralization of the intense smell typical of N-acetylcysteine.

In one embodiment, N-acetylcysteine is of animal origin, obtained according to the methods known in the art.

In another alternative embodiment, the N-acetylcysteine of the present invention is synthetic (sold by A.C.E.F. Spa). Synthetic N-acetylcysteine has the advantage of being less odorous than that of animal origin.

In addition to the described components, the formulation of the invention may contain functional excipients known in the art and used by the person skilled in the art for the preparation of a cream, such as for example: glycerin, lecithin, phytic acid, sodium hydroxide, lactic acid, tocopherol, ascorbyl palmitate, citric acid, alone or, preferably, in mixture with each other.

The excipients are present in a total amount between about 23% and 46% w/w, with values between about 26% and 44% w/w being particularly preferred.

Preferably, the present formulation for topical use is in the form of a cream or a lotion, and preferably has a pH between 5.5 and 6.5, advantageously about 6. The Applicants have indeed found that buffering the pH to such values by neutralizing lactic acid with sodium hydroxide to form sodium lactate, the stability of N-acetylcysteine and urea is improved compared to more acidic or more basic conditions.

The cream formulation not only allows to have a product that is easily applied onto the skin, as it is absorbed quickly, but also allows an application that does not grease, since it is a water-based formulation.

The Applicants have found that this formulation is effective for the treatment of various dermatological diseases, wherein, for example, desquamation, thickening, erythema, swelling and/or loss of elasticity of the dermis are involved.

In fact, the present formulation is suitable for use as a medicament for the prevention and treatment of the diabetic foot syndrome, as an exfoliating and emollient agent. The topical application of this formulation helps to reduce the thickening of the dermis, typical of patients suffering from diabetes, and to soften and elasticise it.

The present formulation is also effective for the treatment and prevention of dermatological disorders caused by the Sudeck syndrome, fighting desquamation and promoting the restoration of an elastic and compact skin environment.

Furthermore, the formulation of the invention is also effective in the treatment and prevention of dermatological disorders caused by chemotherapy, for example palmar-plantar erythrodysesthesia, making the desquamated skin more compact and moisturized and soothing the intense erythema, thanks to its moisturizing properties.

Dermatological disorders from radiation therapy can also be effectively treated with the formulation of the present invention. In fact, the formulation acts as an emollient and antioxidant, thus reducing the symptoms of radiodermatitis, and thus allowing to proceed with the radiotherapy treatment without suffering from the associated dermatological symptoms.

Further characteristics and the advantages of the present invention will become clear from the description of some embodiments given below by way of illustration and not of limitation.

### Example 1

### Formulation with 1.5% of N-acetylcysteine

In one embodiment, the maintenance formulation of the invention consists of an oily phase and an aqueous phase comprising 1.5% of N-acetylcysteine by weight with respect to the total weight of the composition.

The formulation is defined as follows:

| **Substance** | **Function** | **Percentage (%) w/w** |
|---|---|---|
| Cetearyl alcohol | Consistency factor | 7.9 |
| Cetearyl glucoside | Emulsifier | 1.1 |
| Glyceryl stearate | Consistency factor | 1.0 |
| Ethylhexyl palmitate | Emollient | 5.0 |
| Cyclopentasiloxane | Emollient | 2.1 |
| Dimethiconol | Texturizing agent | 0.4 |
| Dimethicone | Defoamer | 0.5 |
| Lecithin | Emulsifier | 0.02 |
| Tocopherol | Antioxidant | 0.004 |
| Ascorbyl palmitate | Antioxidant | 0.003 |
| Oily phase | | |
| Citric acid | pH Adjuster | 0.0007 |
| Glycerin | Humectant | 3.0 |
| Benzyl alcohol | Preservative | 0.9 |
| Dehydroacetic acid | Preservative | 0.1 |
| Sodium phytate | Sequestrant | 0.1 |
| Lactic acid | pH Adjuster | 1.8 |
| Sodium hydroxyde | pH Adjuster | 1.27 |
| **Urea** | **Functional substance** | **7.0** |
| Potassium cetyl phosphate | Emulsifier | 0.3 |
| **N-acetylcysteine** | **Functional substance** | **1.5** |
| Xanthan gum | Rheology modifier | 0.3 |
| Perfume | Perfume | 0.6 |
| Water | Solvent | to 100 |
| Aqueous layer | | |

### Preparation procedure

The above defined formulation is obtained according to the following preparation procedure.

In a turbo-emulsifier, the substances of the aqueous phase are added except for the preservatives, the perfume, the sequestrant (sodium phytate), urea and N-acetylcysteine. The temperature of the turbo-emulsifier is then brought up to 78-80 °C.

Upon reaching the indicated temperature, N-acetylcysteine, previously dissolved in a portion of production water, and urea are then added.

In a fuser, the substances of the oily phase, except for cyclopentasiloxane and tocopherol, are heated to 80 °C.

After checking that the fats are melted, the oily phase is drawn into the aqueous one by means of a vacuum pump by operating the turbine (2000-2500 rpm) and the stirrers (20-40 rpm) for about 10 minutes.

The mixture is then allowed to cool leaving only the stirrers (20-40 rpm) operated; when the temperature of the mixture is lower than 60 °C, cyclopentasiloxane and tocopherol are added, then the turbine (2000-2500 rpm) is re-operated for 5-10 minutes.

At a temperature below 40 °C, preservatives, perfume and preservative are added. The formulation of the invention thus obtained is further left to cool down to room temperature, while it is constantly mixed by the action of the stirrers.

N-acetylcysteine can be substituted with a dispersion (sold by KALIS Srl) of N-acetylcysteine in carboxymethylcellulose, characterized by a strong reduction of the intense smell typical of N-acetylcysteine.

N-acetylcysteine may be of animal or non-animal origin; in the latter case, the product sold by A.C.E.F. spa (CAS number 616-91-1) is used.

### Example 2

### First formulation with 3% of N-acetylcysteine

In another embodiment, the formulation of the invention consists of an oily phase and an aqueous phase comprising 3% of N-acetylcysteine by weight with respect to the total weight of the formulation.

The formulation is defined as follows:

| **Substance** | **Function** | **Percentage (%) P/P** |
|---|---|---|
| Cetearyl alcohol | Consistency factor | 3.0 |
| PEG-8 ester of C₁₂₋₂₀ acids | Emulsifier | 11.0 |
| Glyceryl stearate | Consistency factor | 1.5 |
| Ethylhexyl Palmitate | Emollient | 5.0 |
| Olus Oil | Emollient | 10.0 |
| Dimethicone | Defoamer | 0.5 |
| Lecithin | Emulsifier | 0.03 |
| Tocopherol | Antioxidant | 0.006 |
| Ascorbyl palmitate | Antioxidant | 0.005 |
| Oily phase | | |
| Citric acid | pH Adjuster | 0.0007 |
| Glycerin | Humectant | 4.0 |
| Benzyl alcohol | Preservative | 0.85 |
| Dehydroacetic acid | Preservative | 0.1 |
| Phytanyc acid | Sequestrant | 0.05 |
| Lactic acid | pH Adjuster | 4.5 |
| Sodium hydroxide | pH Adjuster | 2.72 |
| **Urea** | **Functional substance** | **20.0** |
| **N-acetylcysteine** | **Functional substance** | **3.0** |
| Xanthan gum | Rheology modifier | 0.5 |
| Perfume | Perfume | 0.65 |
| Water | Solvent | to 100 |
| Aqueous phase | | |

### Preparation procedure

The above defined formulation is obtained according to the preparation method described in Example 1, except that only tocopherol is not inserted into the fuser for the preparation of the oily phase; in fact, tocopherol is added when the mixture has reached a temperature lower than 60 °C.

### Example 3

### Second formulation with 3% of N-acetylcysteine

In a further embodiment, the present invention provides a formulation comprising 3% of N-acetylcysteine, differing from the formulation of Example 2 in that it also contains the bark extract of *Mimosa tenuiflora,* tocopherol acetate and panthenol.

| **Substance** | **Function** | **Percentage (%) p/p** |
|---|---|---|
| PEG-8 ester of C₁₂₋₂₀ acids | Emulsifier | 11.00 |
| Cetearyl alcohol | Consistency factor | 2.75 |
| Glyceryl stearate | Consistency factor | 1.50 |
| Olus oil | Emollient | 7.50 |
| Ethylhexyl palmitate | Emollient | 5.00 |
| Dimethicone | Defoamer | 0.50 |
| Tocopheryl-acetate | Antioxidant | 2.00 |
| Lecithin | Emulsifier | 0.03 |
| Tocopherol | Antioxidant | 0.01 |
| Ascorbyl palmitate | Antioxidant | 0.01 |
| Oily phase | | |
| Citric acid | pH Adjuster | 0.01 |
| Glycerin | Humectant | 3.60 |
| Benzyl alcohol | Preservative | 0.80 |
| Dehydroacetic acid | Preservative | 0.08 |
| Phytanyc acid | Sequestrant | 0.05 |
| Lactic acid | pH Adjuster | 4.50 |
| Sodium hydroxide | pH Adjuster | 2.40 |
| **Urea** | **Functional substance** | **20.00** |
| **N-acetylcysteine** | **Functional substance** | **3.00** |
| Bark extract of *Mimosa tenuiflora* | Cicatrizing/moisturizing/lenitive ingredient | 0.18 |
| Panthenol | Cicatrizing/lenitive/ filmogenic ingredient | 0.75 |
| Perfume | Perfume | 0.80 |
| Xanthan gum | Rheology modifier | 0.50 |
| Water | Solvent | to 100 |
| Aqueous phase | | |

### Preparation procedure

The process for preparing the above-defined formulation is the same as described in Example 1.

### Example 4

### Formulation with N-acetylcysteine complexed with beta-cyclodextrins

In a last embodiment, the present invention provides a formulation comprising 3% of synthetic N-acetylcysteine complexed with 2-hydroxypropyl-beta-ciclodextrins, provided by I.R.A. ISTITUTO RICERCHE APPLIED SRL with the trade name of CYCLOSYSTEM COMPLEX N-ACETILCISTEINA IN HP-BETA-CDX (product code 9011).

The formulation is defined as follows:

| **Substance** | **Function** | **Percentage (%) w/w** |
|---|---|---|
| PEG-8 ester of C₁₂₋₂₀ acids | Emulsifier | 11.00 |
| Cetearyl alcohol | Consistency factor | 2.75 |
| Glyceryl stearate | Consistency factor | 1.50 |
| Olus oil | Emollient | 7.50 |
| Ethylhexyl Palmitate | Emollient | 5.00 |
| Dimethicone | Defoamer | 0.50 |
| Tocopheryl acetate | Antioxidant | 2.00 |
| Lecithin | Emulsifier | 0.03 |
| Tocopherol | Antioxidant | 0.01 |
| Ascorbyl palmitate | Antioxidant | 0.01 |
| Oily phase | | |
| Citric acid | pH Adjuster | 0.01 |
| Glycerin | Humectant | 3.60 |
| Benzyl alcohol | Preservative | 0.80 |
| Dehydroacetic acid | Preservative | 0.08 |
| Phytanyc acid | Sequestrant | 0.05 |
| Lactic acid | pH Adjuster | 4.50 |
| Sodium hydroxide | pH Adjuster | 2.40 |
| **Urea** | **Functional substance** | **20.00** |
| **CYCLOSYSTEM COMPLEX** consisting of: Acetyl-cysteine (3%) 2-hydroxypropyl-betacyclodextrin (4%) | **Functional substance** | **7.00** |
| Bark extract of *Mimosa tenuiflora* | Cicatrizer | 0.18 |
| Panthenol | Cicatrizer | 0.75 |
| Perfume | Perfume | 0.80 |
| Xanthan gum | Rheology modifier | 0.50 |
| Water | Solvent | to 100 |
| Aqueous phase | | |

### Preparation procedure

The preparation process of the formulation as defined above involves the fusion of the oily portion, comprising all the above-mentioned substances, except for the aforesaid CYCLOSYSTEM COMPLEX product, and the preparation of the aqueous phase as described in Example 1.

The CYCLOSYSTEM COMPLEX product is added at the end of the process and mixed with the other substances until the final formulation is cooled to room temperature.

### Example 5

*Treatment of a dermatological disorder caused by radiotherapy by topical application of the formulation of the present invention.*

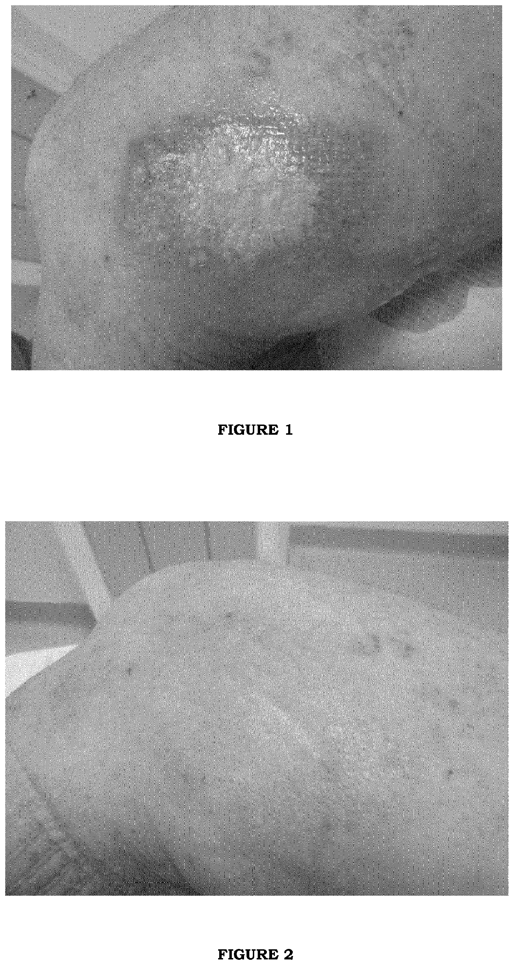

Figure 1 shows a skin area located at the thigh level of a patient who underwent radiotherapy cycles of 9 MeV, for a 45 Gy (Grey) dose in 9 fractions with a 0.5 cm bolus. The patient started treatment in the last sessions of radiotherapy and was then subjected to treatment with the formulation of the invention for 15 days, applying it twice a day.

The skin shows symptoms of radiodermatitis, characterized by inflammation in the treated areas with redness, irritation, desquamation and blisters.

Figure 2 shows the same area after treatment with the formulation according to Example 2: it is evident that the skin shows clear signs of healing: blisters are no longer present, as well as the signs of desquamation and irritation, the skin appears more compact, soft and elastic.

### Example 6

*Treatment of the diabetic foot syndrome by topical application of the formulation of the present invention.*

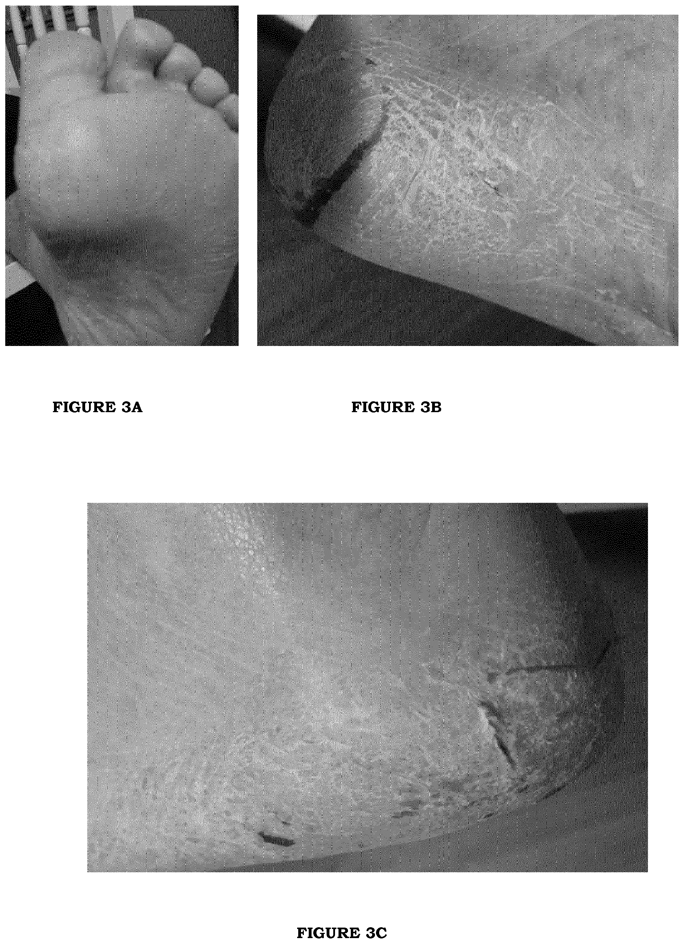

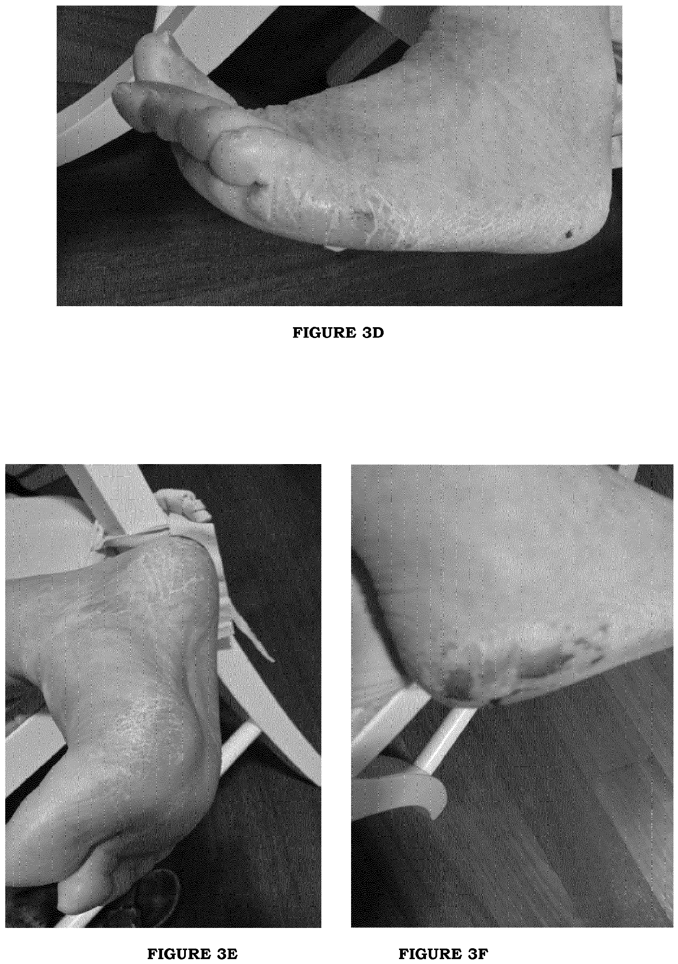

Figures 3A-3F show the symptoms of the diabetic foot syndrome: chapping, tearing in the thinnest parts of the foot, thickening and swelling in the parts of the foot wherein the dermis has stiffened.

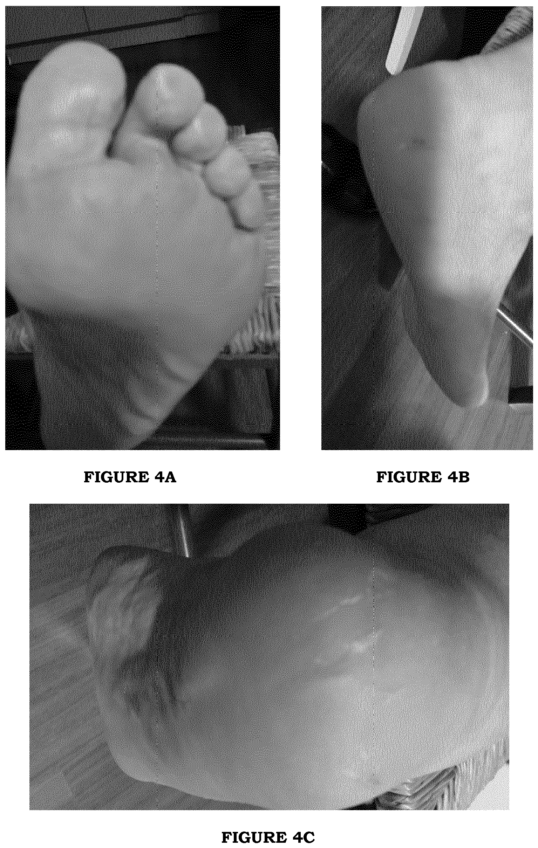

Figures 4A-4C show the effects of the application of the formulation according to Example 2 of the invention, for two weeks.

The skin treated with the formulation for 14 days does not show any chaps or tears, and it is also possible to appreciate a significant thinning of the dermis in the affected areas; moreover, it appears to be compact, uniform and elastic.

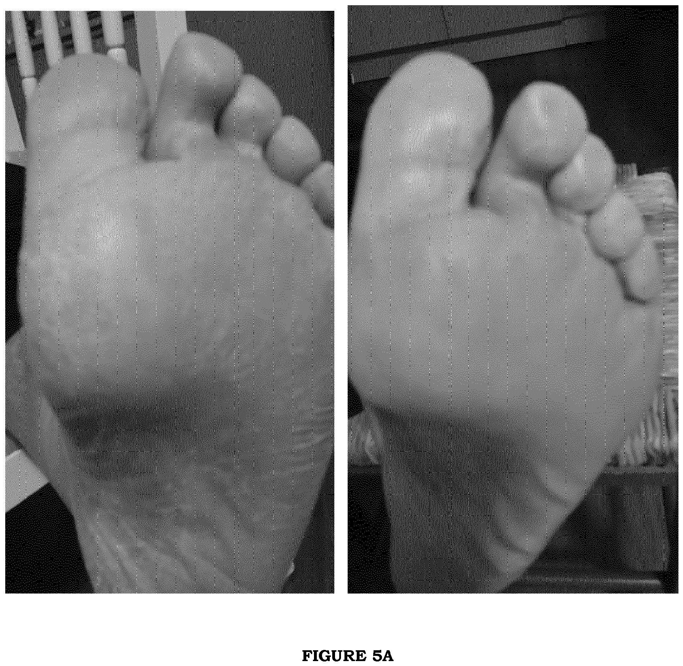

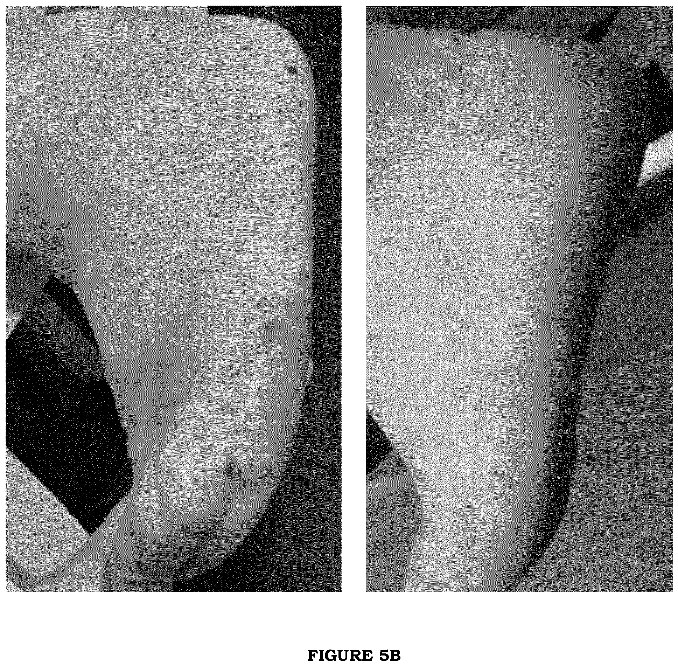

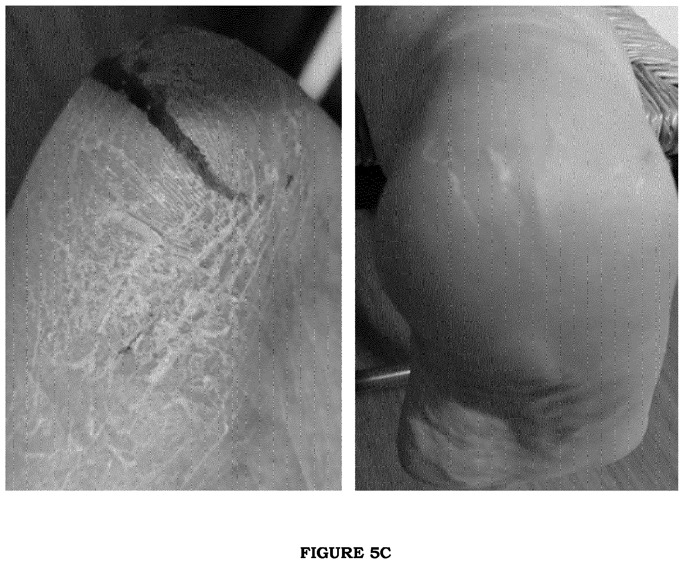

Figures 5A-5C compare the diabetic foot before (left) and after (right) the treatment with the formulation of the invention to more easily appreciate the beneficial effects of the topical application of the formulation of the present invention.

### Example 7

*Treatment of dermatological disorders of the Sudeck syndrome by topical application of the formulation of the present invention.*

The formulation according to Example 2 was applied 3-4 times a day, at the onset of the first burning symptoms, up to 6 times a day, on the skin of a patient suffering from Sudeck syndrome.

In particular, this application for 10 days resulted in complete healing of the treated skin region: the sores were stretched, the skin appeared more elastic and compact and the burning felt by the patient was reduced to complete cancellation.

## Claims

1. A formulation for topical application comprising, in percentages by weight of the total weight of the formulation, 0.5%-10% of N-acetylcysteine and 2-30% of urea and a dermatologically acceptable vehicle, for use in the treatment and/or prevention of a condition selected from the group consisting of dermatological disorders of the Sudeck syndrome, chemotherapy-induced palmar-plantar erythrodysesthesia, dermatological disorders caused by radiotherapy and diabetic foot syndrome.

2. The formulation for the use according to claim 1, comprising 1-4% of N-acetylcysteine and 5-25% of urea.

3. The formulation for the use according to claim 1, comprising 1.5-3% of N-acetylcysteine and 7-20% of urea.

4. The formulation for the use according to any one of claims 1 to 3, wherein said N-acetylcysteine is in form of a complex with beta-cyclodextrins.

5. The formulation for the use according to claim 4, wherein said beta-cyclodextrins are 2-hydroxypropyl-beta-cyclodextrins.

6. The formulation for the use according to any one of claims 1 to 5, wherein said N-acetylcysteine is of synthetic origin.

7. The formulation for the use according to any one of claims 1 to 3, comprising, in percentages by weight of the total weight of the formulation:
| | |
|---|---|
| Water | 29-65 |
| Urea | 7-20 |
| Cetearyl alcohol | 2.75-8.0 |
| Ethylhexyl palmitate | 4.0-6.0 |
| Glycerin | 3.0-4.0 |
| Lactic acid | 1.8-4.5 |
| N-acetylcysteine | 1.5-3.0 |
| Sodium hydroxide | 1.2-2.8 |
| Glyceryl stearate | 1.0-1.5 |
| Benzyl alcohol | 0.8-0.9 |
| Perfume | 0.6-0.8 |
| Dimethicone | 0.4-0.6 |
| Xanthan gum | 0.3-0.5 |
| Dehydroacetic acid | 0.08-0.1 |
| Lecithin | 0.02-0.03 |
| Tocopherol | 0.004-0.1 |
| Ascorbyl palmitate | 0.003-0.01 |
| Citric acid | 0.0007-0.01 |

8. The formulation for the use according to any one of claims 1 to 5, comprising, in percentages by weight of the total weight of the formulation:
| | |
|---|---|
| Water | 29-65 |
| Urea | 7-20 |
| Cetearyl alcohol | 2.75-8.0 |
| Ethylhexyl palmitate | 4.0-6.0 |
| Glycerin | 3.0-4.0 |
| Lactic acid | 1.8-4.5 |
| N-acetylcysteine | 1.5-3.0 |
| Beta-cyclodextrins | 3.5-4.5 |
| Sodium hydroxide | 1.2-2.8 |
| Glyceryl stearate | 1.0-1.5 |
| Benzyl alcohol | 0.8-0.9 |
| Perfume | 0.6-0.8 |
| Dimethicone | 0.4-0.6 |
| Xanthan gum | 0.3-0.5 |
| Dehydroacetic acid | 0.08-0.1 |
| Lecithin | 0.02-0.03 |
| Tocopherol | 0.004-0.1 |
| Ascorbyl palmitate | 0.003-0.01 |
| Citric acid | 0.0007-0.01 |

9. The formulation for the use according to any one of claims 1-8, having a pH between 5.5 and 6.5.

10. The formulation for the use according to claim 9, having a pH of about 6.

11. The formulation for the use according to any one of claims 1 to 10, wherein such formulation is in form of a cream or a lotion.

## Patentansprüche

1. Formulierung zur topischen Anwendung, enthaltend in Gewichtsprozent des Gesamtgewichts der Formulierung 0,5-10 % N-Acetylcystein und 2-30 % Harnstoff und einen dermatologisch akzeptablen Träger, zur Verwendung bei der Behandlung und/oder Vorbeugung eines Zustands, der aus der Gruppe bestehend aus dermatologischen Störungen des Sudeck-Syndroms, Chemotherapie-induzierter palmar-plantarer Erythrodysästhesie, durch Strahlentherapie verursachten dermatologischen Störungen und diabetischem Fußsyndrom ausgewählt ist.

2. Formulierung zur Verwendung nach Anspruch 1, enthaltend 1-4% N-Acetylcystein und 5-25% Harnstoff.

3. Formulierung zur Verwendung nach Anspruch 1, enthaltend 1,5-3% N-Acetylcystein und 7-20% Harnstoff.

4. Formulierung zur Verwendung nach einem der Ansprüche 1 bis 3, wobei das N-Acetylcystein in Form eines Komplexes mit beta-Cyclodextrinen vorliegt.

5. Formulierung zur Verwendung nach Anspruch 4, wobei die beta-Cyclodextrine 2-Hydroxypropyl-beta-Cyclodextrine sind.

6. Formulierung zur Verwendung nach einem der Ansprüche 1 bis 5, wobei das N-Acetylcystein synthetischen Ursprungs ist.

7. Formulierung zur Verwendung nach einem der Ansprüche 1 bis 3, enthaltend in Gewichtsprozent des Gesamtgewichts der Formulierung:
| | | |
|---|---|---|
| | Wasser | 29-65 |
| | Harnstoff | 7-20 |
| Cetearylalkohol | | 2,75-8,0 |
| Ethylhexylpalmitat | | 4,0-6,0 |
| | Glycerin | 3,0-4,0 |
| | Milchsäure | 1,8-4,5 |
| | N-Acetylcystein | 1,5-3,0 |
| | Natriumhydroxid | 1,2-2,8 |
| | Glycerinstearat | 1,0-1,5 |
| | Benzylalkohol | 0,8-0,9 |
| | Parfüm | 0,6-0,8 |
| | Dimethicon | 0,4-0,6 |
| | Xanthangummi | 0,3-0,5 |
| Dehydroessigsäure | | 0,08-0,1 |
| | Lecithin | 0,02-0,03 |
| | Tocopherol | 0,004-0,1 |
| | Ascorbylpalmitat | 0,003-0,01 |
| Zitronensäure | | 0,0007-0,01 |

8. Formulierung zur Verwendung nach einem der Ansprüche 1 bis 5, enthaltend in Gewichtsprozent des Gesamtgewichts der Formulierung:
| | | |
|---|---|---|
| Wasser | | 29-65 |
| Harnstoff | | 7-20 |
| Cetearylalkohol | | 2,75-8,0 |
| Ethylhexylpalmitat | | 4,0-6,0 |
| Glycerin | | 3,0-4,0 |
| Milchsäure | | 1,8-4,5 |
| N-Acetylcystein | | 1,5-3,0 |
| Beta-Cyclodextrine | | 3,5-4,5 |
| Natriumhydroxid | | 1,2-2,8 |
| Glycerinstearat | | 1,0-1,5 |
| Benzylalkohol | | 0,8-0,9 |
| Parfüm | | 0,6-0,8 |
| Dimethicon | | 0,4-0,6 |
| Xanthangummi | | 0,3-0,5 |
| Dehydroessigsäure | | 0,08-0,1 |
| | Lecithin | 0,02-0,03 |
| | Tocopherol | 0,004-0,1 |
| | Ascorbylpalmitat | 0,003-0,01 |
| Zitronensäure | | 0,0007-0,01 |

9. Formulierung zur Verwendung nach einem der Ansprüche 1-8, aufweisend einen pH-Wert zwischen 5,5 und 6,5.

10. Formulierung zur Verwendung nach Anspruch 9, aufweisend einen pH-Wert von etwa 6.

11. Formulierung zur Verwendung nach einem der Ansprüche 1 bis 10, wobei die Formulierung in Form einer Creme oder Lotion vorliegt.

## Revendications

1. Formulation pour une application topique comprenant, en pourcentages en poids du poids total de la formulation, 0,5 %-10 % de N-acétylcystéine et 2-30 % d'urée et un véhicule dermatologiquement acceptable, pour une utilisation dans le traitement et/ou la prévention d'une pathologie choisie dans le groupe constitué par des troubles dermatologiques de la maladie de Sudeck, l'érythrodysesthésie palmo-plantaire induite par une chimiothérapie, des troubles dermatologiques causés par une radiothérapie et le syndrome du pied diabétique.

2. Formulation pour une utilisation selon la revendication 1, comprenant 1-4 % de N-acétylcystéine et 5-25 % d'urée.

3. Formulation pour une utilisation selon la revendication 1, comprenant 1,5-3 % de N-acétylcystéine et 7-20 % d'urée.

4. Formulation pour une utilisation selon l'une quelconque des revendications 1 à 3, dans laquelle ladite N-acétylcystéine est sous une forme d'un complexe avec des bêta-cyclodextrines.

5. Formulation pour une utilisation selon la revendication 4, dans laquelle lesdites bêta-cyclodextrines sont des 2-hydroxypropyl-bêta-cyclodextrines.

6. Formulation pour une utilisation selon l'une quelconque des revendications 1 à 5, dans laquelle ladite N-acétylcystéine est d'origine synthétique.

7. Formulation pour une utilisation selon l'une quelconque des revendications 1 à 3, comprenant, en pourcentages en poids du poids total de la formulation :
| | |
|---|---|
| Eau | 29-65 |
| Urée | 7-20 |
| Alcool cétéarylique | 2,75-8,0 |
| Palmitate d'éthylhexyle | 4,0-6,0 |
| Glycérine | 3,0-4,0 |
| Acide lactique | 1,8-4,5 |
| N-acétylcystéine | 1,5-3,0 |
| Hydroxyde de sodium | 1,2-2,8 |
| Stéarate de glycérol | 1,0-1,5 |
| Alcool benzylique | 0,8-0,9 |
| Parfum | 0,6-0,8 |
| Diméthicone | 0,4-0,6 |
| Gomme xanthane | 0,3-0,5 |
| Acide déhydroacétique | 0,08-0,1 |
| Lécithine | 0,02-0,03 |
| Tocophérol | 0,004-0,1 |
| Palmitate d'ascorbyle | 0,003-0,01 |
| Acide citrique | 0,0007-0,01 |

8. Formulation pour une utilisation selon l'une quelconque des revendications 1 à 5, comprenant, en pourcentages en poids du poids total de la formulation :
| | |
|---|---|
| Eau | 29-65 |
| Urée | 7-20 |
| Alcool cétéarylique | 2,75-8,0 |
| Palmitate d'éthylhexyle | 4,0-6,0 |
| Glycérine | 3,0-4,0 |
| Acide lactique | 1,8-4,5 |
| N-acétylcystéine | 1,5-3,0 |
| Bêta-cyclodextrines | 3,5-4,5 |
| Hydroxyde de sodium | 1,2-2,8 |
| Stéarate de glycérol | 1,0-1,5 |
| Alcool benzylique | 0,8-0,9 |
| Parfum | 0,6-0,8 |
| Diméthicone | 0,4-0,6 |
| Gomme xanthane | 0,3-0,5 |
| Acide déhydroacétique | 0,08-0,1 |
| Lécithine | 0,02-0,03 |
| Tocophérol | 0,004-0,1 |
| Palmitate d'ascorbyle | 0,003-0,01 |
| Acide citrique | 0,0007-0,01 |

9. Formulation pour une utilisation selon l'une quelconque des revendications 1-8, ayant un pH compris entre 5,5 et 6,5.

10. Formulation pour une utilisation selon la revendication 9, ayant un pH d'environ 6.

11. Formulation pour une utilisation selon l'une quelconque des revendications 1 à 10, dans laquelle une telle formulation est sous une forme de crème ou de lotion.
